# EUROPÄISCHE PATENTANMELDUNG

(11) **EP 0 999 277 A1**
(43) Veröffentlichungstag der Anmeldung: **10.05.2000**
(21) Anmeldenummer: 98121243.4
(22) Anmeldetag: 07.11.1998
(51) Int. Cl.: C12N 15/70, C12N 1/38, C12N 15/62, C12P 21/02

(54) **Verfahren zur Expression rekombinanter Proteine unter Ausnutzung der Wirkung von Stress und Stressantwortmechanismen in Gegenwart kompatibler Solute**

(71) Anmelder: Barth, Stefan, Dr. rer. nat., 50931 Köln (DE)
(72) Erfinder: Barth, Stefan, Dr.Dr., 50674 Köln (DE); Engert, Andreas, Dr., 50672 Köln (DE); Louis, Petra, 53117 Bonn (DE); Galinski, Erwin, Prof.Dr., 53127 Bonn (DE); Huhn, Michael, Dr., 51375 Leverkusen (DE)
(74) Vertreter: Meyers, Hans-Wilhelm, Dr.Dipl.-Chem.

(57) **Zusammenfassung**

Verfahren zur Expression und Gewinnung rekombinanter Proteine durch transformierte Organismen in Kulturen, dadurch gekennzeichnet, daß die transformierten Organismen durch Änderung der Umgebungsbedingungen in einen Streßzustand versetzt werden und die Produktion des rekombinanten Proteins in Gegenwart von kompatiblen Soluten durch einen Induktor ausgelöst wird.

## Beschreibung

Gegenstand der vorliegenden Erfindung ist ein Verfahren zur Expression rekombinanter Proteine unter Streßbedingungen und anschließender Gewinnung der Produkte.

Ein Ziel der industriellen Biotechnologie auf Basis der Molekularbiologie ist die Produktion von Substanzen, die durch chemische Totalsynthese - wenn überhaupt - nur äußerst schwierig zu erhalten sind. Dazu gehören insbesondere hochmolekulare, biologisch oder pharmazeutisch wirksame Stoffe, wie beispielsweise Fette, Kohlenhydrate, Vitamine sowie Peptide oder Proteine, beispielsweise Antikörper, hochmolekulare heterogene Konjugate und Fusionsproteine (z.B. Immuntoxine, Zytokinfusionsproteine, Diabodies), aber auch niedermolekulare Substanzen. Durch gentechnologische Methoden können in der Regel gentechnisch veränderte Organismen hergestellt werden, die entweder in ihr Genom integrierte oder extrachromosomale, genetisch veränderte Informationen tragen. Als die üblicherweise in Betracht kommenden Systeme haben sich prokaryotische Zellen, insbesondere *E.coli*-Bakterien, aber auch Hefen, die als nicht-typische eukaryotische Zellen über einen komplexeren Stoffwechselapparat verfügen, sowie Insekten- und Säugetierzellen etabliert.

Die zur Transformation zu verwendenden Strukturen sind nach dem Stand der Technik konstruierte Nukleotidsequenzen, die beispielsweise für Promotoren, Signalsequenzen, die erwünschten Zielsequenzen, Markergene und/oder ähnliche Produkte kodieren. Die Expression kann gegebenenenfalls verstärkt werden durch Kombination mit Silencer-, Enhancersequenzen und/oder Transkriptionsfaktoren und/oder Chaperonen.

Verschiedene Faktoren müssen von einem Expressionssystem erfüllt werden, um eine 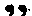 high level"-Produktion von rekombinanten Proteinen zu gewährleisten. Hier zu nennen sind Zellwachstumscharakteristika, der Expressionslevel, intra- und extrazelluläre Modifikationen und die biologische Aktivität des Zielproteins.

Es konnte zwar vielfach gezeigt werden, daß *E.coli* sich für die Expression großer Mengen rekombinanter Proteine besonders eignet, jedoch nicht jedes eingebrachte Gen effizient in diesem System exprimiert werden kann.

Die größten Einschränkungen bezüglich der funktionellen Bildung verschiedener eukaryotischer Proteine liegen in der verminderten Fähigkeit der Bakterien, posttranslationale Modifikationen durchzuführen, der Abwesenheit eines effizienten Sekretionsmechanismus zur Ausschleusung der Proteine ins Kulturmedium, sowie in der verminderten Fähigkeit, Disulfidbrücken zu bilden.

In Gram-negativen Bakterien sind zwei Bildungsräume (das Periplasma und das Zytoplasma) für die Produktion rekombinanter Proteine beschrieben.

Für die zytoplasmatische Expression rekombinanter Proteine müssen bestimmte Parameter beachtet werden: einerseits tritt hier eine verstärkte Proteindegradation auf, da *E.coli* im Zytoplasma die höchste Anzahl von Proteasen aufweist, andererseits ist mit Schwierigkeiten bei der Reinigung des rekombinanten Zielproteins aus dem großen Pool der intrazellulär vorhandenen Proteine zu rechnen. Oft liegt das Zielprotein nicht in seiner nativen Form, sondern als Einschlußkörperchen ( 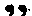 inclusion bodies") vor. Nach der Gewinnung der Einschlußkörperchen muß das rekombinante Protein durch aufwendige De- und Renaturierungstechniken in ein funktionelles Protein umgewandelt werden.

Ein zusätzliches Problem stellt das Reduktionspotential des natürlich vorhandenen zytoplasmatischen Redox-Systems dar. Bakterielle zytoplasmatische Proteine enthalten wenig Cysteinreste und Disulfidbrücken. Viele *E.coli*-Proteine, die über stabile Disulfidbrücken verfügen, werden aus dem Zytoplasma ins Periplasma transportiert, so daß Proteine aus Säugern, die sich als komplexe tertiäre Strukturen darstellen, im Periplasma zur korrekten Konformation gefaltet werden können.

Im Gegensatz zum zytoplasmatischen Kompartiment weist das Periplasma nur ca. 4% des Gesamtproteingehalts bzw. ungefähr 100 verschiedene Proteine auf, so daß das eingebrachte Zielprotein konzentriert und mit vergleichsweise geringer Kontamination von Fremdproteinen vorliegt. Außerdem ermöglicht die oxidierende Umgebung des Periplasmas eine korrekte Faltung des Proteins (sofern daran Disulfidbrücken beteiligt sind). Für den Transport des Zielproteins durch die innere Membran ins Periplasma muß eine entsprechende Signalsequenz vorhanden sein.

Die vorliegende Erfindung hat sich die Aufgabe gestellt, ein Expressionsverfahren für rekombinante Proteine zur Verfügung zu stellen, bei denen die rekombinanten Proteine gegenüber dem Stand der Technik in höheren Ausbeuten erhalten werden.

Gelöst wird die Aufgabe durch ein Expressionsverfahren mit den Merkmalen des Patentanspruchs 1. Die sich daran anschließenden abhängigen Patentansprüche stellen bevorzugte Ausführungsformen des erfindungsgemäßen Verfahrens dar.

Die erhöhte Ausbeute kann dadurch erreicht werden, daß a) die Expression erhöht wird, b) die Bildung von 'Inclusion Bodies' vermieden wird, c) ein höherer Anteil korrekt gefaltet vorliegt, d) der Anteil an Verunreinigungen mit Fremdprotein verringert wird und e) Verluste durch die Aktivität von Proteasen verringert werden.

Das erfindungsgemäße Verfahren zur Expression rekombinanter Proteine durch transformierte Organismen in Kulturen ist dadurch gekennzeichnet, daß die transformierten Organismen durch Änderung der Umgebungsbedingungen in einen Streßzustand versetzt werden und die Produktion des rekombinanten Proteins in Gegenwart von kompatiblen Soluten durch einen Induktor ausgelöst wird.

Das erfindungsgemäße Verfahren nutzt physiologische Veränderungen des Produktionsstammes aufgrund von Streßbedingungen und Anpassung an diese Bedingungen für die Erhöhung der Ausbeute und/oder korrekte Faltung und/oder Stabilisierung und/oder Aufreinigung von rekombinanten Proteinen.

Die Induktion kann durch den Streß selbst erfolgen, oder durch chemische oder physikalische (z.B. thermische) Auslöser.

"Streß" im Sinne dieser Anmeldung sind nicht-optimale Umweltbedingungen für den Produktionsstamm und "Streßinduktion" die Veränderung eines (oder mehrerer) Umweltparameter(s), die dazu führen, daß der Organismus in irgendeiner Weise darauf reagiert; "Streßzustand" bedeutet den durch Streßinduktion erhaltenen Zustand des Organismus.

Bei sich vermehrenden Organismen führt ein Streßzustand in der Regel dazu, daß das Wachstum zumindest vorübergehend zunächst verlangsamt (und evtl. sogar ganz eingestellt) wird und bei dazu befähigen Organismen eine physiologische Anpassung an die veränderten Umweltbedingungen erfolgt. Abgesehen davon können auch physiologische und/oder strukturelle Veränderungen erfolgen (z.B. Aktivierung von Reparaturmechanismen), die nicht offensichtlich und u.U., wie z.B. bei ruhenden Zellen, nicht an einer morphologischen Veränderung erkennbar sind.

Der Streßzustand im erfindungsgemäßen Verfahren kann durch Änderungen der chemischen oder physikalischen Umgebungsbedingungen erfolgen. Bevorzugt wird der Streßzustand durch eine hohe Zucker- und/oder Polyol- und/oder Salzkonzentration im Kulturmedium erreicht. Weitere Beispiele für Streßfaktoren sind Temperaturerhöhung oder -erniedrigung, UV-Strahlung, für den Organismus unphysiologisch hohe oder niedrige pH-Werte, erhöhte bzw. erniedrigte Osmolarität und/oder Ionenstärke, organische Lösungsmittel etc.

Die Anwendung dieser Streßfaktoren, insbesondere osmotischer Schock, führt bei streßtoleranten oder -resistenten Organismen bzw. solchen, die zur Anpassung befähigt sind, zu charakteristischen Veränderungen und gegebenenfalls zur Synthese und/oder Akkumulation von Streßschutzstoffen. Insbesondere ist für den Fall des osmotischen Stresses bekannt, daß zur Aufrechterhaltung der Wachstums- und Lebensbedingungen kompatible Solute gebildet oder, falls vorhanden, dem umgebenden Medium entzogen werden (Galinski und Trüper, 1994). Kompatible Solute (organische Osmotika) sind als niedermolekulare Naturstoffe definiert, die den Stoffwechsel ansonsten auch in hoher Konzentration nicht oder nur wenig beeinflussen (Brown, 1976; Galinski 1995). Neben dem rein osmotischen Effekt kommt den kompatiblen Soluten im Zytoplasma auch eine protektive Bedeutung zu, denn sie müssen die Hydratation bzw. Solubilität des Enzymapparates sicherstellen (Galinski, 1995; Lippert und Galinski, 1992).

Im erfindungsgemäßen Verfahren erfolgt die Induktion in Gegenwart kompatibler Solute. Zu dieser Stoffgruppe gehören Zucker und Polyole (z.B. Trehalose, Glycerin, Glycosylglycerin), natürliche Aminosäuren (z.B. Alanin, Prolin, Glutamin), Derivate von Aminosäuren (z.B. N-acetylierte Diaminosäuren, Glutamin-1-Amide, Taurin), Betaine (z.B. Glycinbetain) und Ectoine (L-Ectoin, S,S-β-Hydroxyectoin). Dabei können die kompatiblen Solute sowohl von außen zugesetzt werden, als auch von den gentechnisch veränderten Organismen der Kultur gebildet werden.

Es wird bevorzugt, daß eine Kultur der erfindungsgemäß einzusetzenden Organismen eine hohe Zelldichte aufweist, insbesondere in Schüttelkulturen eine optische Dichte OD₆₀₀ >2, in Fermentationsanlagen 3-400, vorzugsweise größer 2,5.

Als transformierte Organismen können bevorzugt Bakterien, Hefen, Pilze, Insektenzellen, Tierzellen oder Pflanzenzellen gewählt werden. Als transformierte Organismen werden besonders bevorzugt Gram-negative Bakterien und am stärksten bevorzugt *E.coli*-Bakterien eingesetzt.

Die für die zu synthetisierenden Substanzen codierenden Regionen können auf einem Expressionsplasmid vorhanden sein oder stabil ins Wirtsgenom integriert sein.

Es wird insbesondere bevorzugt, daß das rekombinante Protein eine Leitsequenz für die Ausschleusung des Proteins aus dem Zytoplasma enthält.

Das rekombinante Protein steht unter der Kontrolle eines Promotors. Hier können je nach Expressionssystem starke und schwache Promotoren verwendet werden; insbesondere sind der lac-, der tac- oder der T7-Promotor oder osmotisch regulierbare Promotoren bevorzugt.

Als Expressionsvektor kann insbesondere ein Plasmid verwendet werden, wobei Derivate des pBM1.1 besonders bevorzugt sind. Dieses Plasmid ist in der DE-A-196 48 108 beschrieben.

Das exprimierte rekombinante Protein des erfindungsgemäßen Verfahrens kann bevorzugt ein chimäres Protein, am stärksten bevorzugt ein Immuntoxin sein. Immuntoxine bestehen aus einer Bindungs- und einer Toxindomäne, wobei insbesondere tumorspezifische Bindungsdomänen verwendet werden. Solche Immuntoxine können Tumorzellen selektiv zerstören. Prinzipiell können aber beliebige Proteine, beispielsweise Enzyme, Antikörperfragmente, Peptidhormone oder Cytokine exprimiert werden.

In einer bevorzugten Ausführungsform des erfindungsgemäßen Verfahrens wird die Expression des kompatiblen Stabilisators über ein cotransformiertes Plasmid gesteuert.

Es wird insbesondere bevorzugt, daß das kotransformierte Plasmid die Ectoingene (ectA, ectB, ectC), gegebenenfalls in Kombination mit dem eigenen osmoregulierten oder einem anderen induzierbaren Promoter aufweist. Das Plasmid kann ein pOSM-Derivat mit den Ectoingenen sein. Jedoch kann die Expressionskassette für die Synthese des kompatiblen Stabilisators auch zusätzlich auf einem pBM1.1-Derivat codiert sein.

Insbesondere wird bevorzugt, daß die transformierten Organismen, die das erfindungsgemäße Verfahren durchlaufen haben, nach der Expression aufgeschlossen werden und die Proteine isoliert werden. Der Aufschluß kann insbesondere durch Verbringung der transformierten Organismen in Umgebungsbedingungen mit geringer Salz- und Zuckerkonzentration erfolgen. Bevorzugt wird ein periplasmatischer Aufschluß durchgeführt.

Bevorzugterweise findet die Expression des rekombinanten Proteins bei Temperaturen statt, die unterhalb der optimalen Wachstumstemperatur des transformierten Organismus liegt. Für *E.coli* eignen sich Temperaturen von 15-28°C, besonders bevorzugt 26°C, da bei Temperaturen <35°C ein veränderter Stoffwechsel mit reduzierter Proteaseaktivität in *E.coli* beobachtet worden ist. Der Aufschluß erfolgt jedoch bevorzugt bei einer Temperatur von 4°C (geringste Proteaseaktivität) durch Ultraschall und/oder Freneh-Press (Wechsel von Hochdruck und Normaldruck) oder durch Einfrieren und Auftauen.

Im Anschluß an die Expression und den Aufschluß werden die rekombinanten Proteine durch chromatographische Verfahren bis zur Homogenität gereinigt. Dabei können die für die Trennung der rekombinanten Proteine von Fremdproteinen an sich bekannte Methoden wie Ionenaustauscher- und/oder Metallchelat- und/oder Molekulargewichtschromatographie (Gelfiltration) eingesetzt werden.

Das erfindungsgemäße Verfahren eignet sich insbesondere zur gentechnischen Herstellung von Substanzen, z.B. von rekombinanten Immuntoxinen oder zur Herstellung rekombinanter chimärer Proteine mit mehreren Bindungsfunktionen (Zytokinfusionsproteine, Diabodies).

Im folgenden soll an einem Verfahrensbeispiel gezeigt werden, wie sich die Anwendung eines osmotischen Stresses auf die Expression und Reinigung rekombinanter Proteine auswirkt.

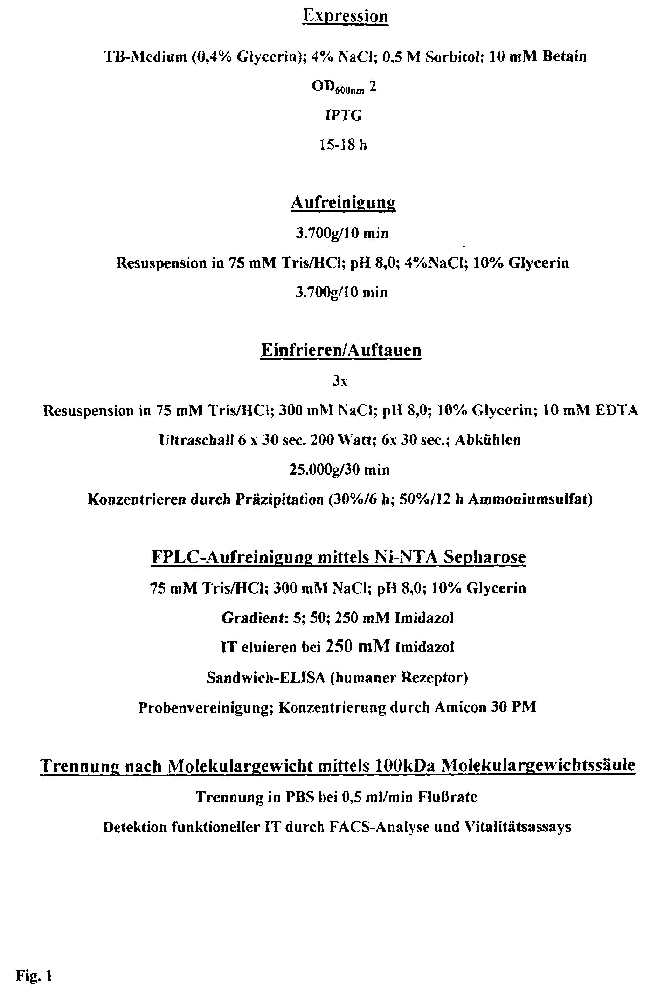

Figur 1 zeigt die Kultivierung, Expression und Reinigung am Beispiel von Ki-4(scFv)-ETA'.

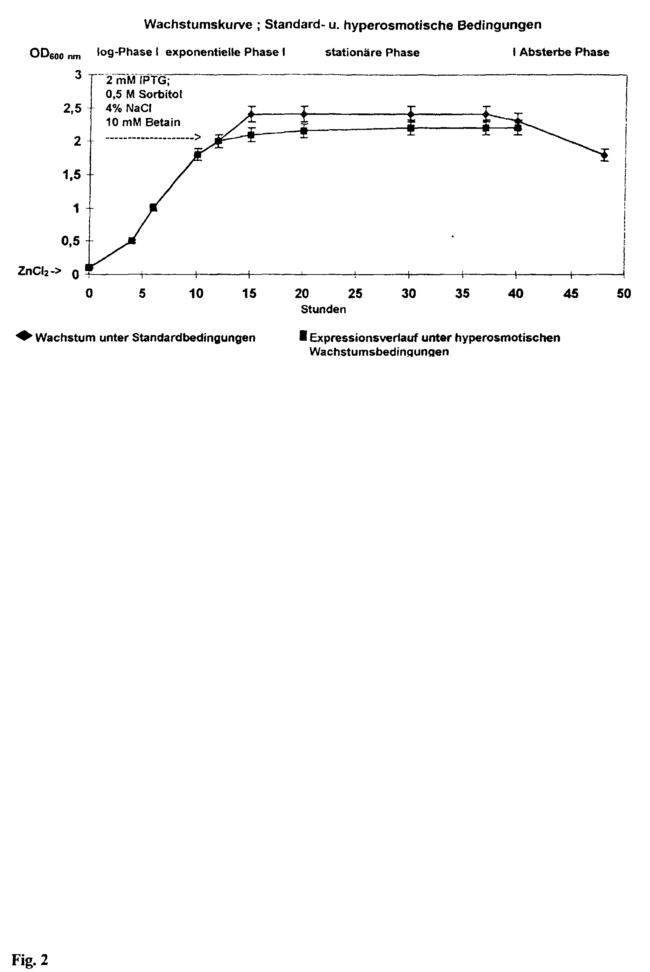

Figur 2 zeigt die Wachstums- und Expressionswerte unter Standardbedingungen (Barth et al. 1998) bzw. bei Verwendung von TB-Medium unter hyperosmotischen Streß (4% NaCl; 0,5 M Sorbitol; 0,5 mM ZnCl₂; 0,4% Glycerin; 10 mM Glycin-Betain) bei gleichzeitiger Expression von Ki-4(scFv)-ETA' durch 2 mM IPTG.

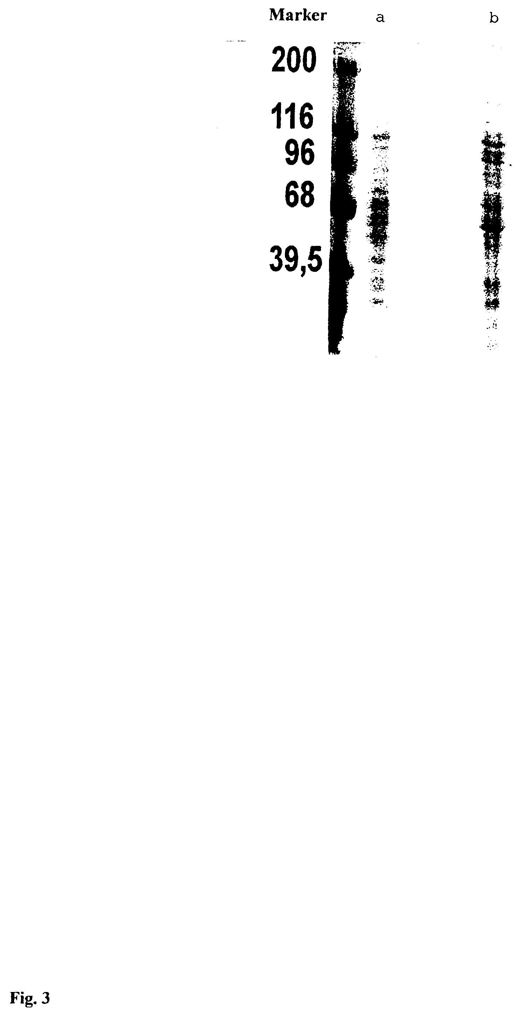

Figur 3 zeigt den Vergleich zweier Kulturansätze. a) Wachstum und Expression von bei Ki-4(scFv)ETA' unter Standardbedingungen (Barth et al., 1998) und b) bei hyperosmotischem Streß (4% NaCl; 0,5 M Sorbitol; 0,5 mM ZnCl₂; 10 mM Glycin-Betain). Visualisierung des Gesamtproteinmusters mittels 10-15% SDS-PAGE (Coomassie gefärbt). M = Marker.

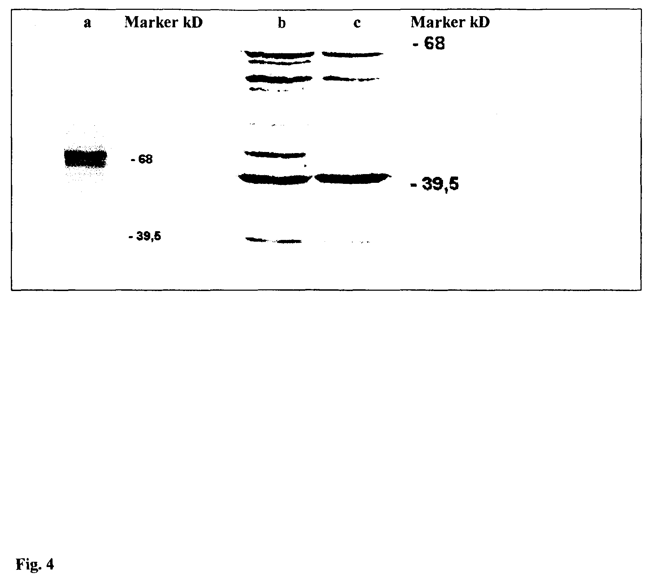

Figur 4 zeigt den Vergleich unterschiedlicher Kulturbedingungen. Ki-4(scFv)ETA' und Fragmente wurde mittels TC-1 Antikörper visualisiert. Grundlage war eine 10% SDS-PAGE mit anschließendem Immunostaining auf Nylonmembran. kD = Kilo Dalton; a) Wachstum und Expression von Ki-4(scFv)ETA' unter hyperosmotischem Streß (4% NaCl; 0,5 M Sorbitol; 0,5 mM ZnCl₂; 10 mM Glycin-Betain); b) bei Standardbedingungen (Barth et al., 1998) und c) unter Standardbedingungen und Zusatz von 0,5 mM ZnCl₂.

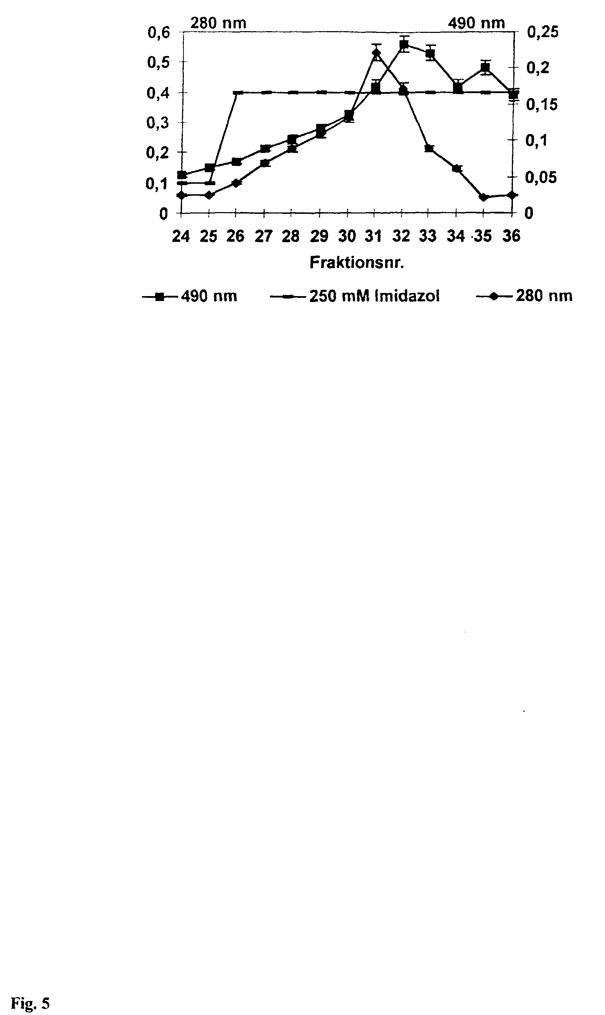

Figur 5 zeigt das Chromatogramm der Aufreinigung des 66 kDa-großen Ki-4(scFv)-ETA' mit Hilfe einer Ni-NTA Sepharose. Das IT wurde bei Zugabe von 250 mM Imidazol eluiert. Die Selektion von Ki-4(scFv)-ETA' positiven Fraktionen wurde durch die Detektion an rCD30 Rezeptor (Sandwich ELISA 490 nm; Barth et al. 1998) durchgeführt.

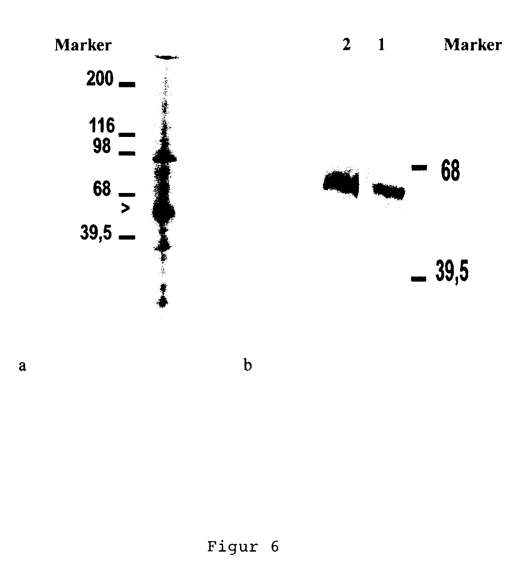

Figur 6 zeigt einen Westernblot einer mit 250 mM Imidazol eluierten Probe von Ki-4(scFv)-ETA'. a) biotinyliertes Protein (Boehringer Mannheim) und b) mittels TC1 (muriner anti-ETA' moab) visualisiert; 1 = Fraktion 35; 2 = gepoolte Fraktion.

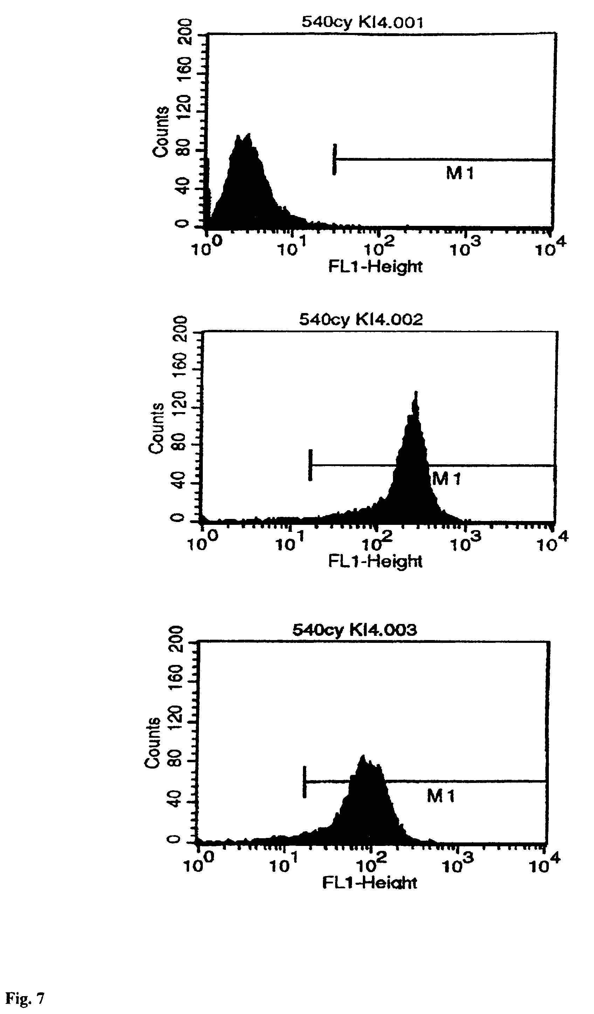

Figur 7 zeigt die spezifische Zellbindungsaktivität von Ki-4(scFv)ETA' mittels FACS-Analyse. L540Cy Zellen wurden mit a) PBS; b) Ki-4(moab); c) Ki-4(scFv)ETA' für 1 Std. bei 4°C inkubiert. Die Zellen wurden mit Maus-anti-ETA (TC-1) und Ziege-anti-Maus FITC-konjugierten Antikörper gefärbt. Die Immunofluoreszenz (FL1 chanel) wurde durchflußzytometrisch durch Verwendung eines FACstar (Beoton Dickinson, USA) gemessen.

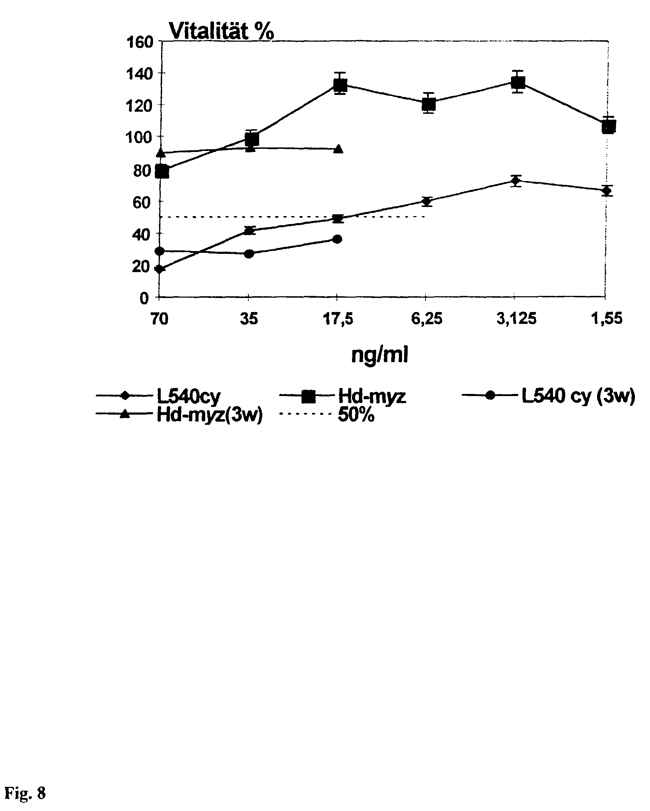

Figur 8 zeigt die Inhibition der Proteinbiosynthese: Nach der Inkubation mit Ki-4(scFv)-ETA' bzw. Ki-4(scFv)-ETA' (3w) für 24 Stunden, wurde [³H]Leucin für 17 Stunden auf die Zellen gegeben. Die Aufnahmerate von [³H]Leucin wurde mittels des Scintillationssystems LS 6500 (Beckman, Germany) gemessen.

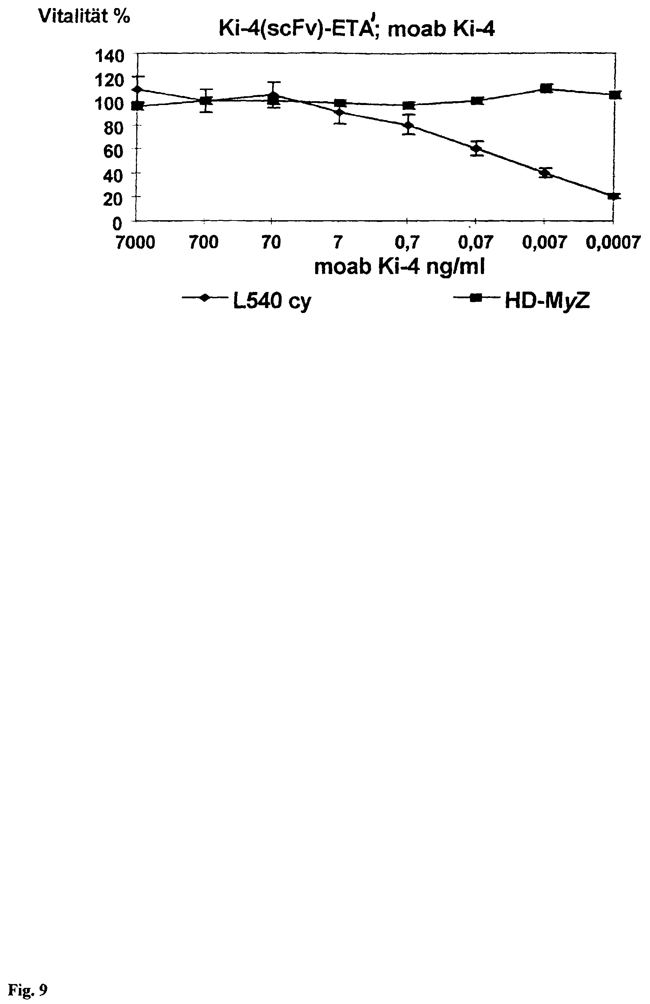

Figur 9 zeigt die Inhibition der Proteinbiosynthese: Darstellung der Kompetition der Zytotoxizität gegenüber L540Cy Zellen. Nach Inkubation mit Ki-4(scFv)-ETA' und Ki-4 moab für 24 Stunden, erfolgte die gleiche Behandlung wie in Fig. 4.

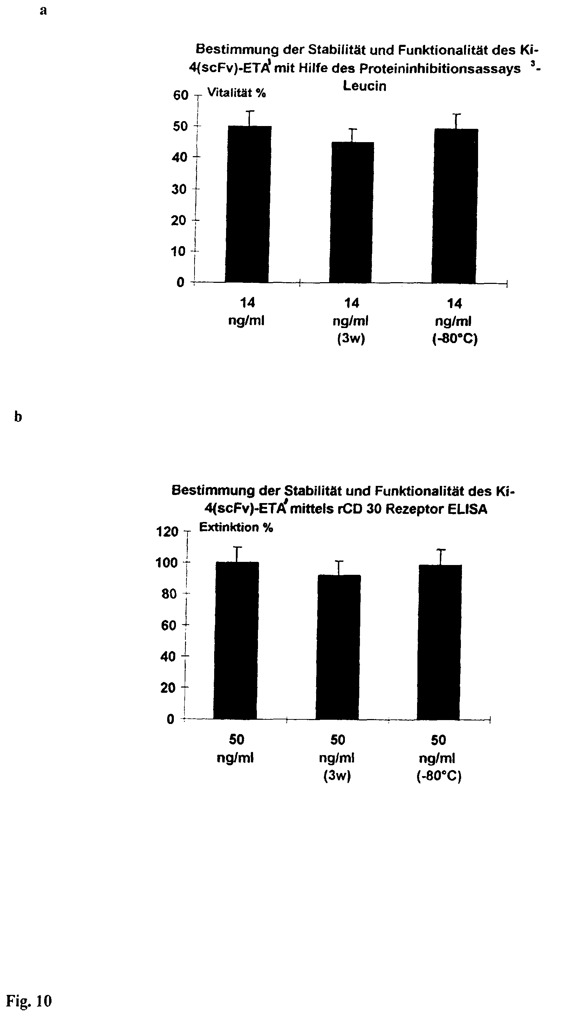

Figur 10 vergleicht die Stabilität und Funktionalität des Ki-4(scFv)-ETA' direkt nach der Gewinnung und Lagerung des IT in PBS für 3 Wochen mit Hilfe von [³H]Leucin-Aufnahmeassays (a) bzw. mittels rCD 30 Rezeptor ELISA (b).

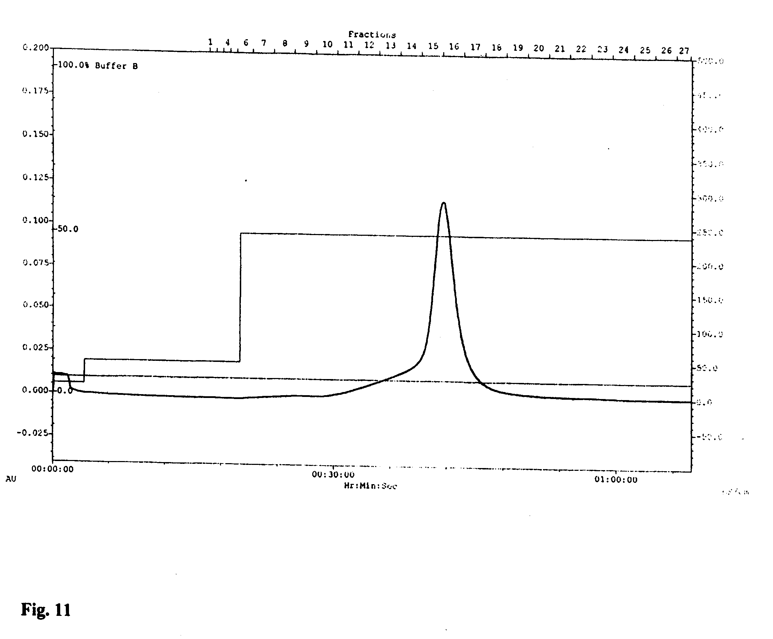

Figur 11: Aufreinigung des 68 kDa-großen RFT5-(scFv)-ETA' mittels Ni-NTA Sepharose. Das Immuntoxin wurde unter Zugabe von 250 mM Imidazol eluiert. Die positiven 250 mM Imidazol-Fraktionen wurden zur Vorbereitung für die Molekulargewichtstrennung gepoolt und mittels Ultrafiltration an AMICON 30 auf 750 µl eingeengt.

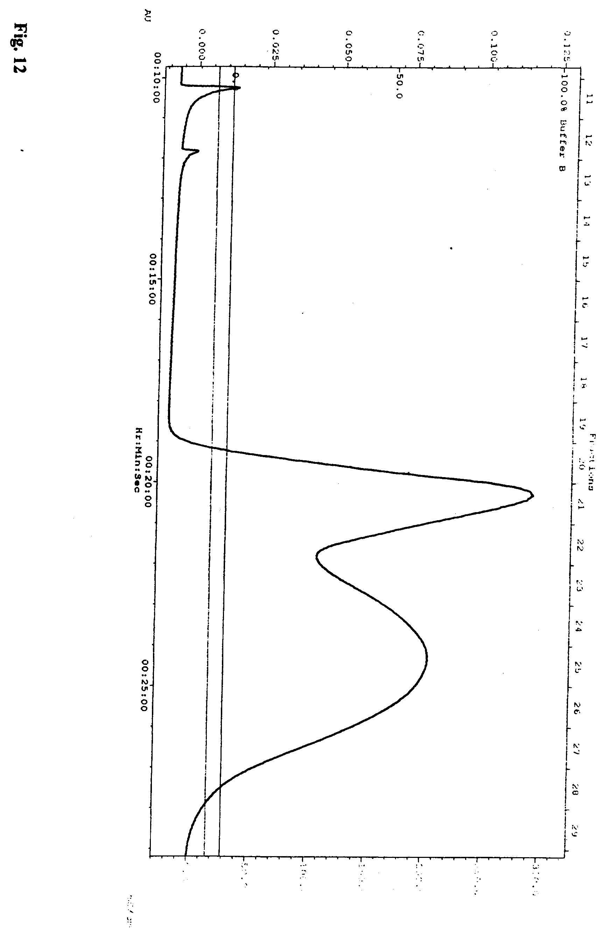

Figur 12: Aufreinigung des 68 kDa-großen RFT5-(scFv)-ETA' mit Hilfe einer 100 kDa Molekulargewichtssäule. Das Immuntoxin wurde im Molekulargewichtsbereich von <100 kDa - 20 kDa von hochmolekularen Aggregaten bzw. niedrigmolekularen Proteinen getrennt. Die Selektion von RFTS-(scFv)-ETA' positiven Fraktionen wurde durch die Detektion an rCD25 Rezeptor (Sandwich ELISA (490 nm); Barth et al. 1998) und über den Nachweis spezifischer Zellbindungsaktivität mittels FACS-Analyse durchgeführt.

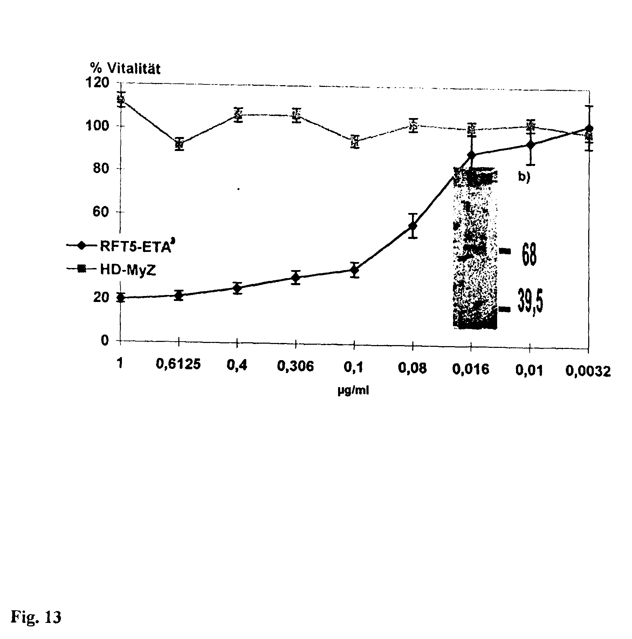

Figur 13: a) Untersuchungen zur Inhibition der Proteinbiosynthese: Nach der Inkubation mit RFT5(scFv)-ETA' (Fraktion 26) für 24 Stunden wurde [³H]Leucin für 17 Stunden auf die Zellen gegeben. Die Aufnahmerate von [³H]Leucin wurde mittels des Scintillationssystems LS 6500 (Beckman, Germany) gemessen.
   b) Darstellung der Fraktion 26 aus der Molekulargewichtatrennung mittels 10% SDS-PAGE und Visualisierung durch Färbung mit Coomassie.

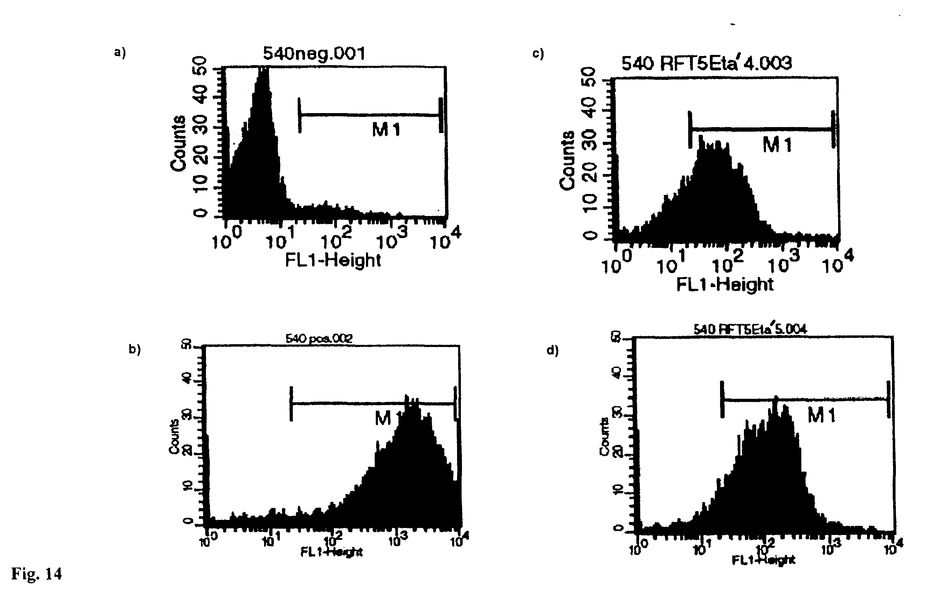

Figur 14: Nachweis der spezifischen Zellbindungsaktivität von RFT5(scFv)ETA' mittels FACS-Analyse. L540Cy-Zellen wurden mit a) PBS, b) RFT5(moab), c) RFT5(scFv)ETA' Fraktion 25, d) RFT5(scFv)ETA' Fraktion 26 für 1 Std. bei 4°C inkubiert. Die Zellen wurden mit Maus-anti-ETA (TC-1) und Ziege-anti-Maus FITC-konjugierten Antikörper gefärbt. Die Immunofluoreszenz (FL1 chanel) wurde durchflußzytometrisch durch Verwendung eines FACStar (Becton Dickinson, USA) gemessen.

### Herstellung rekombinanter anti-CD30 und anti-CD25 Immuntoxine in E.coli BL21(DE3)

### 1. Methoden

### 1.1 Transformation der Organismen zur Expression rekombinanter chimärer Proteine

Bindungsfähige, einzelsträngige variable Fragmente wurden mittels Standardverfahren (Sambrook et al., 1989) in den bakteriellen Expressionsvektor pBM1.1 (Matthey et al., 1998) kloniert und dadurch mit einer Deletionsmutante des *Pseudomonas* Exotoxin A fusioniert. Die Ligationsansätze wurden mit Phenol extrahiert, mit Ethanol präzipitiert und in 10 µl H₂O resuspendiert. 2.5 µl dieser DNA-Lösung wurde nach einem beschriebenen Protokoll (Dower et al., 1988) durch Elektroporation in *E.coli* BL21(DE3) eingebracht. Transformierte Bakterien wurden auf Kanamycinhaltigem Medium (2xLB mit 50 µg Kanamycin/ml und 2% Glukose) kultiviert. Positive Klone wurden durch Restriktionsanalyse verifiziert.

Das pOSM (Louis und Galinski, 1997) mit der Ectoinsynthesekassette wurde ebenfalls zusätzlich mit Standardtechniken in positive Klone eingebracht. Transformierte Bakterien wurden auf Kanamycin- (50 µg/ml) und chloramphenicol-haltigem (20 µg/ml) LB-Medium kultiviert. Positive Klone wurden ebenfalls durch Restriktionsanalyse verifiziert.

### 1.2 Anzucht und Induktion der gentechnisch veränderten Organismen

Transformierte *E.coli* BL21(DE3) wurden in Terrific Broth-Medium [TB] (Sambrook et al., 1989; 1,2% Bacto-Trypton, 2,4% Bacto-Yeast Extract, 0,4 % Glycerin, 0,17 M KH₂PO₄, 0,72 M K₂HPO₄) supplementiert mit 50 µg/ml Kanamycin und 0,5 mM ZnCl₂ für 12-15 h bzw. über Nacht bei 26°C und 200 rpm inkubiert.

Anschließend wurden die Kulturen in 0,5 1-Erlenmeyerkolben mit Schikanen, in einem Volumen von 200 ml TB-Medium überführt und bis zu einer OD₆₀₀ von ≤ 2 angezogen. Anschließend wurde der osmotische Wert des Mediums mit 0,5 M Sorbitol, 4% NaCl heraufgesetzt und als kompatibles Solut 10 mM Betain oder Ectoin hinzugefügt. Beide Substanzen haben vergleichbare Wirkung, wobei allerdings Ectoin über Kotransformation mit Plasmiden der pOSM-Reihe oder durch zusätzliche Kodierung auf dem pBM1.1-Derivat auch direkt von den Zellen synthetisiert werden kann.

Die Kulturen wurden für 15-60 min bei 26°C/200 rpm inkubiert und dann durch Zugabe von 2 mM IPTG die Expression des Targetproteins eingeleitet. Nach Erreichen einer OD₆₀₀ von 2,3-2,4 wurden die Bakterien durch einen Zentrifugationsschritt (3.700 g, 10 mm, 4°C) geerntet. Alle weiteren Schritte wurden auf Eis durchgeführt. Nach einem Waschschritt (Pellet wurde in 75 mM Tris/HCl pH 8, 4% NaCl, 10% Glycerin resupendiert und homogenisiert) mit anschließender Zentrifugation (3.700 g, 10 mm, 4°C) wurde das Gewicht des Naßpellets bestimmt und das Bakterienpellet vor dem Aufschluß bei -80°C bzw. bei -196°C für 10 min schockgefroren.

### 1.3 Periplasma-Aufschluß und Aufreinigung rekombinanter Immuntoxine

Im Anschluß an die Einfrierphase wurde das Bakterienpellet in Ultraschallpuffer (75 mM Tris/HCl pH 8, 300 mM NaCl, 1 Tablette Proteaseinhibitoren [Boeringer]/50 ml, 5 mM DTT, 10 mM EDTA, 10% Glycerol) auf Eis resuspendiert, homogenisiert (15 min) und anschließend 6x30 sec bei 200 Watt Ultraschall-behandelt. Zwischen den einzelnen Inkubationsphasen erfolgte eine gleich lange Abkühlungsphase auf Eis (30 sec). Um Immuntoxin (IT) quantitativ zu gewinnen, wurde das Pellet nochmals bei -80°C bzw. -196°C gefroren und die soeben beschriebenen Arbeitsschritte wurden bis zu 3x wiederholt. Die gewonnenen Fraktionen wurden vereinigt und sterilfiltriert. Nach sequentieller 50% Ammoniumsulfat(AS)-Fällung wurden die Proben in 30% AS für 4-6 h inkubiert, anschließend in 50% AS angereichert und schließlich bei 4°C über Nacht präzipitiert. Zur Gewinnung der Proben wurden diese bei 25.000 g für 45 min zentrifugiert. Das Proteinpellet wurde in Probenpuffer (75 mM Tris/HCl pH 8, 300 mM NaCl, 10% Glycerol) aufgenommen. Danach wurden die Proben bei 4°C mittels Hitrap-Desalting Säule auf einer FPLC Anlage umgepuffert (75 mM Tris/HCl pH 8, 300 mM NaCl, 10% Glyceroll). Im Anschluß an die Entsalzungssäule wurde das Protein in einem ersten Schritt metallchelatchromatographisch über eine Ni-NTA-Säule (Qiagen) aufgetragen.

### 1.4 In-vitro-Charakterisierung der rekombinanten Immuntoxine

Nach Äquilibrierung der Ni-NTA-Säule wurde das aufgearbeitete Protein des Periplasmaaufschlusses zur Trennung störender Fremdproteine auf die Ni-NTA-Säule gegeben. Die Trennung erfolgte mittels einer FPLC-Anlage durch einen Stufengradienten mit 5 mM, 50 mM und 250 mM Imidazol. Funktionelles Immuntoxin ließ sich quantitativ mit 250 mM Imidazol von der Ni-NTA-Säule eluieren. Nach Analyse der 250 mM Imidazol-Fraktionen mittels CD30 Rezeptor-Sandwich-ELISA (siehe oben, Barth et al., 1998; Smith et al. 1993) wurden die IT-Proben vereinigt und durch Ultrafiltration eingeengt. Die auf 750 µl eingeengten IT-Proben wurden anschließend mittels 100 kDa Molekulargewichtssäule (BioRad) von Proteinen > 100 kDa und Proteinaggregaten sowie von Proteinen < 20 kDa getrennt. Zur Bestimmung des Reinheitsgrades der eluierten Proteinfraktionen wurden die Proben biotinyliert (Biotinylierungs-Kit der Firma Boehringer Mannheim, Deutschland) bzw. mittels SDS-PAGE getrennt und über Coomassie-blue-Färbung visualisiert. Die beschriebene Methode ist in Figur 1 als Fließschema dargestellt.

Zur Bestimmung der spezifischen biologischen Aktivität kamen zwei Vitalitätstest zu Einsatz. Einerseits wurde die Aufnahmerate von radioaktivem [³H]-Leucin an Antigen-positiven bzw. -negativen Zellen unter Einfluß des ITs gemessen (Barth et al. 1998). Zum anderen wurden die Atmungsraten durch Messung der Aktivität mitochondrialer Dehydrogenasen unter IT-Einfluß an den entsprechenden Zellinien bestimmt (XTT-Test von Boehringer Mannheim, Deutschland).

### 2. Ergebnisse

### 2.1 Wachstumsverlauf und Expression der rekombinanten Immuntoxine

Salzstreß wurde im letzten Drittel der exponentiellen, bakteriellen Wachstumsphase durch 4% NaCl und 0,5 M Sorbitol induziert. Zur optimalen Bewältigung dieser Streßsituation wurde hier als kompatibles Solut 10 mM Betain oder Ectoin extern zugegeben. Die K⁺-Akkumulation ist in Gegenwart von Betain und Prolin verminden (Dinnbier et al. 1988). Betain wird innerhalb von 10 min ausreichend angereichert (Csonka 1989), dann erfolgt die Einleitung der Expression durch Zugabe von IPTG. Der Organismus wuchs im Zeitraum von 12-40 h um 0,2-0,4 OD₆₀₀-Einheiten (Figur 2). Die Absterbephase setzt nach 48 h ein. Zur Minimierung der Azidität des Mediums wurde alls C-Quelle Glycerol eingesetzt und außerdem mittels Kalium-Phosphat gepuffert (Csonka 1989).

### 2.2 Vergleich der Wachstumsbedingungen und der Expressionseigenschaften

Die Organismen, die IT unter standardisierten Kulturbedingungen exprimierten, zeigten im Proteinbandenmuster deutliche Unterschiede zu Organismen, die im oben beschriebenen Medium unter osmotischem Streß in Kombination mit kompatiblen Soluten angezogen wurden. Klar erkennbare Unterschiede zeigten sich im kDa-Bereich von 40-100 kDa (Figur 3): unter osmotischem Streß traten zusätzliche Banden in diesem Bereich auf Verschiedene Autoren, unter ihnen Hengge-Aronis 1996, beschreiben als einen Erklärungsansatz für dieses Phänomen Umgruppierungen und Neubildungen von Membranbestandteilen (Hengge-Aronis et al.1991; Jung et al. 1989,1990), neusynthetisierte Enzyme und Proteine (Hengge-Aronis et al. 1993, Lange und Hengge-Aronis 1991; Weichart et al.1993; Yim et al. 1994; Yim und Villarejo, 1992), Porine (McCann et al. 1991) und Bildung von Osmolyten und deren Shuttle-Systeme etc.

Der Vergleich der Expressionsqualtitäten unter den verschiedenen Wachstumsbedingungen zeigte deutliche Proteaseaktivität unter Standardbedingungen (Figur 4). Die absolute Menge an gebildetem IT war bei diesen Kulturen höher. Das Wachstum unter ZnCl₂ führte zu einer verminderten Proteaseaktivität während der Expression des IT (Baneyx et al. 1991), da ZnCl₂ Serin-spezifische Proteasen sehr effektiv inhibiert (Sugimura und Hijashi 1988; Baneyx und Georgion 1991a; Bradley et al. 1998). Da der IT-Abbau wahrscheinlich eine Reaktion des Organismus auf verstärkt synthetisiertes *Pseudomonas*-Exotoxin darstellt, liegen fragmentierte Proteine vor, die gleiche Aufreinigungsstrukturen aufweisen, wie das komplette, funktionelle Fusionstoxin. Daraus resultiert eine aufwendigerere Aufreinigungsstrategie, um störende nicht-funktionelle IT-Bestandteile abzutrennen. Diese Proteinfragmente zeigen ein gleiches Verhalten bei AS-Fällung, der Molekulargewichtschromatographie, sowie bei der Metall-Chelat-Chromatographie.

### 2.3 Aufreinigung mittels Ni-NTA-Säule

Unter Ausnutzung der Aufreinigungsmöglichkeiten über ein vorhandene His-Tag konnten die Fusionstoxine weitestgehend von Fremdprotein getrennt werden (Figur 5, 6, 11). Immuntoxine wurden mit 250 mM Imidazol eluiert und konnten mittels Rezeptor-Sandwich-ELISA (hier CD30) weitgehend quantitativ detektiert werden (Figur 5, Fraktionen 30-36).

### 2.4 Aufreinigung unter Verwendung einer 100kDa Molekularsäule

Zur weiteren Aufreinigung der IT wurde das Proteingemisch nach ihren Molekulargewichten getrennt. Hierzu wurde eine 100kDa Molekularsäule von Biorad eingesetzt. Mit Hilfe dieser Säule konnten Proteine >100 kDa und Proteinaggregate bzw. Proteine < 20 kDa von den IT getrennt werden. Die Fraktionen 21-27 der Figur 12 enthielten RFT5(scFv)-ETA', wobei der höchste Reinigungsgrad auf die Fraktionen 25-26 fallen. Hier zeigte sich eine singuläre Bande nach Anfärbung mittels Coomassie Blue (Figur 13b).

### 2.5 Funktionalität der IT

Zur Bestimmung der Funktionalität wurde die Proteinsynthese von Antigenpositiven bzw. -negativen Zellinien unter dem Einfluß der IT ermittelt. Zur Bestimmung der Funktionalität wurde exemplarisch die Fraktion 35 aus der vorherig beschriebenen Ni-NTA-Aufreinigung (Figur 5) verwendet. Die Bindungsaktivität der rekombinanten Ki-4(scFv)-ETA' bzw. RFT5(scFv)-ETA' wurde durch ELISA- und FACS-Analysen (Figur 7, 14) und eine spezifische Inhibition der Proteinbiosynthese durch [³H]Leucin-Aufhahmeassays (Figur 8, 13a) bestätigt.

Zur Kompetition der Bindung (Figur 9) und der daraus resultierenden spezifischen, zytotoxischen Wirksamkeit des Ki-4(scFv)-ETA' im Toxizitätstest wurde der Ursprungsantikörper des Ki-4(scFv)-ETA' eingesetzt, ein anti-CD30-Maus Antikörper. Hierbei wurde die Konzentration des Maus-anti-CD30 Antikörpers in unterschiedlichen Konzentrationen eingesetzt, die Konzentration des rekombinanten chimären Ki-4(scFv)-ETA' blieb gleich. Deutlich zu erkennen ist die zunehmende spezifische Zytotoxizität des Ki-4(scFv)-ETA' mit Abnahme der Konzentration des Maus-anti-CD30 Antikörpers.

### 2.6 Stabilität

Zur Ermittlung der Stabilität der Konstrukte (Figur 8, 10) wurden die Proben in PBS bzw. Medium für 3 Wochen bei 4°C aufbewahrt und anschließend deren Toxizitäten verglichen. Hier zeigte sich eine maximale Abweichung von höchstens 10% (Figur 8, 10).

Es stellte sich heraus (Figur 6), daß die IT auch in einem Gemisch aus 10% Glycerol, 75 mM Tris/HCl pH 8, 10 mM Betain und 300 mM NaCl eingefroren werden können. Hier zeigten sich keine signifikanten Funktionseinschränkungen (<<10%).

### Literatur

BANEYX, F., GEORGIOU G. (1991). Construction and characterization of *Escherichia coli* strains deficient in multiple secreted proteases: protease III degrades high molecular weight substrates *in vivo*. J. Bacteriol. 173: 2696-2703
BARTH, S., HUHN, M., WELS, W., DIEHL, V., ENGERT, A. (1998). Construction and *in vitro* evaluation of RFT5(scFv)-ETA', a new recombinant single-chain immunotoxin with specific cytotoxicity toward CD25⁺ Hodgkin-derived cell lines. Int. J. Molec. Med. 1: 249-256
BROWN, A.B. (1976). Microbial water stress. Bact. Rev. 40: 803-846
CSONKA, L.N. (1989). Physiological and genetic responses fo bacteria to osmotic stress. Microbiol. Rev. 53: 121-147
DINNBIER, U, LIMPINSEL, E., SCHMID R., BAKKER E. P.(1988). Transient accumulation of potassium glutamate and ist replacement by trehalose during adaption of growing cells of *Escherichia coli* K-12 to elevated sodium chloride concentrations. Arch. Microbiol. 150: 348-357
DOWER, W.J., MILLER, J.F., RAGSDALE, C.W. (1988). High affinity transformation of *E.coli* by high voltage electroporation. Nucleic Acids Res. 16: 6127-6145
GALINSKI, E.A. (1995). Osmoadaptation in bacteria. Adv. Microbial Physiol. 37: 273-328
GALINSKI, E.A., TRÜPER, H.G. (1994). Microbial behaviour in salt-stressed ecosystems. FEMS Microbiology Reviews 15: 95-108
HENGGE-ARONIS, R. (1996). Back to log phase: sigma S as a global regulator in the osmotic control of gene expression in *Escherichia coli*. Mol. Microbiol. 21 (5): 887-893
HENGGE-ARONIS, R., KLEIN, W., LANGE, R., RIMMELE, M., BOSS, W. (1991). Trehalose synthesis genes are controlled by the putative sigma factor encoded by rpoS and are involved in stationary-phase thermotolerance in *Escherichia coli*. J. Bacteriol. 173: 7918-7924
HENGGE-ARONIS, R., LANGE, R., HENNEBERG, N., FISCHER, D. (1993). Osmotic regulation of rpoS-dependent genes in *Escherichia coli*. J. Bacteriol. 175: 259-265
HORN-LORENS, O., TIEMANN, M., LANGE, H., KOBARG, J., HAFNER, M., HANSEN, H., STERRY, W., PARWARESCH, R.M., LEMKE, H. (1995). Shedding of the soluble form of CD30 from Hodgkin-analogous cell line L540 is strongly inhibited by a new CD30-specific antibody (Ki-4). Int. J. Cancer 60: 539-544
JUNG, J. U., GUTIERREZ, C., MARTIN,F., ARDOUREL, M., VILLAREJO, M (1990).Transcription of osmB, a gene encoding an *Escherichia coli* lipoprotein, is regulated by signals. J. Biol. Chem. 265: 10574-10581
JUNG, J. U., GUTIERREZ, C., VILLAREJO, M (1989). Sequence of an osmotically inducible lipoprotein gene. J Bacteriol. 171: 511-520
KATZ, B.A., CLARK, J.M., FINER-MOORE, J.S., JENKINS, T.E., JOHNSON, C.R., ROSS, M.J., LUONG, C., MOORE, W.R., STROUD, R.M (1998). Design of potent selective zinc-mediated sehne protease inhibitors. Nature 391: 608-611
LANGE, R., HENGGE-ARONIS, R. (1991). Identification of a central regulator of stationary-phase gene expression in *Escherichia coli*. Mol. Microbiol. 5 (1): 49-59
LIPPERT, K., GALINSKI, E.A. (1992). Enzyme stabilization by ectoine-type compatible solutes: protection against heating, freezing and drying. Appl. Microbiol. Biotechnol. 37: 61-65
LOUIS, P., GALINSKI, E.A. (1997). Characterization of genes for the biosynthesis of the compatible solute ectoine from *Marinococcus halophilus* and osmoregulated expression in *Escherichia coli*. Microbiol. 143: 1141-1149.
LUARD, E.J. (1983). Activity of isocitrate dehydrogenase from three filamentous fungi in relation to osmotic and solute effects. Arch. Microbiol. 134: 233-237
MATTHEY, B., KLIMKA, A., DIEHL, V., ENGERT, A., BARTH, S. (1998) New pET-based vectors for high efficiency expression of recombinant ETA'-based fusion toxins. submitted
MCCANN, M.P., KIDWELL, J.P., MATIN, A (1991). The putative sigma factor katf has a central role in development of stravation-mediated general resistance in *Escherichia coli*. J. Bacteriol. 173: 4188-4194
MEASURES, J.C. (1975). Role of amino acids in osmoregulation of nonhalophilic bacteria. Nature. 257: 398-400
RICHEY, B., CAYLEY, D.S., MOSSING, M., KOLKA, C., ANDERSON, C.F., FARRAR, C.T. RECORD, M.T. JR., (1987). Variability in the intracellular ionic environment o*f Escherichia coli*: differences between *in vitro* and *in vivo* effects of ion concentrations on protein-DNA intractions and gene expression. J. Biol. Chem. 262: 7157-7164
SAMBROOK, J., FRITSCH, E.F., MANIATIS, T. (1989) Molecular cloning. A laboratory manual. second edition. Cold Spring Harbor Laboratory Press
SMITH, C.A, GRUSS, H.J., ANDERSON, D.T., FARRAH, T., BAKER, E., SUTHERLAND, R.G., BRUNNAN, C.J., COPERLAND, N., G., JENKINS, N. A., .GRABSTEIN, K.H., GUNIAK, B., MCALISTER, I.L., FANSLOW, W., ALDERSON, M., FALK B., GIMPEL, S., GILLIS S., DIN W.S., GOODWIN, R.G., ARMITAGE, R.J. (1993). CD30 antigen, a marker for Hodgkin's lymphoma, is a receptor whose ligand defines an emerging family of cytokines with homology to TNF. Cell 73: 1349-1360
SUGIMURA, K., HIGASHI, N. (1988). A novel outer membrane-associated protease in *Escherichia coli*. Proc. Natl. Acad. Sci. USA 79: 1830-1833
WEICHART, D., LANGE, R., HENNEBERG, N., HENGGE-ARONIS, R., (1993). Identification and characterisation of stationary phase-induce genes in *Escherichia coli*. Mol. Microbiol. 10: 407-420
YIM, H.H., BREMS, R.L., VILLAREJO, M. (1994). Molecular characterization of the promoter of osmy, an rpoS dependent gene. J. Bacteriol. 176: 100-107
YIM, H.H., VILLAREJO (1992). OsmyY, a new hyperosmotically inducible gene, encodes a periplasmic protein in *Escherichia coli*. J. Bacteriol. 174: 3637-3644

## Patentansprüche

1. Verfahren zur Expression und Gewinnung rekombinanter Proteine durch transformierte Organismen in Kulturen, dadurch gekennzeichnet, daß die transformierten Organismen durch Änderung der Umgebungsbedingungen in einen Streßzustand versetzt werden und die Produktion des rekombinanten Proteins in Gegenwart von kompatiblen Soluten durch einen Induktor ausgelöst wird.

2. Verfahren gemäß Anspruch 1, dadurch gekennzeichnet, daß der Streßzustand durch Änderung der chemischen oder physikalischen Umgebungsbedingungen erfolgt, wie durch Strahlung und/oder Temperaturerhöhung, insbesondere durch Erhöhung des osmotischen Wertes im Kulturmedium durch gelöste Stoffe, wie durch eine hohe Salz-, Zucker- oder Polyolkonzentration bzw. Kombination verschiedener gut löslicher Substanzen.

3. Verfahren gemäß mindestens einem der Ansprüche 1 und/oder 2, dadurch gekennzeichnet, daß die kompatiblen Solute aus der Gruppe der Zucker und Polyole (wie Trehalose, Glycerin, Glycosylglycerin), natürlichen Aminosäuren (wie Alanin, Prolin, Glutamin), Derivate von Aminosäuren (wie N-acetylierte Diaminosäuren, Glutamin-1-Amide, Taurin), Betaine (wie Glycinbetain) und Ectoine (wie L-Ectoin, S,S-β-Hydroxyectoin) gewählt werden.

4. Verfahren gemäß mindestens einem der Ansprüche 1 bis 3, dadurch gekennzeichnet, daß die kompatiblen Solute der Kultur zugesetzt werden und/oder von den transformierten Organismen selbst gebildet werden.

5. Verfahren gemäß mindestens einem der Ansprüche 1 bis 4, dadurch gekennzeichnet, daß die Kultur eine hohe Zelldichte aufweist.

6. Verfahren gemäß mindestens einem der Ansprüche 1 bis 5, dadurch gekennzeichnet, daß die transformierten Organismen aus der Gruppe der Bakterien, Hefen, Pilze, Säuger-, Insekten- oder Pflanzenzellen gewählt werden.

7. Verfahren gemäß mindestens einem der Ansprüche 1 bis 6, dadurch gekennzeichnet, daß die codierenden Regionen der zu synthetisierenden Substanzen auf einem Expressionsvektor vorhanden oder stabil ins Genom integriert sind.

8. Verfahren gemäß mindestens einem der Ansprüche 1 bis 7, dadurch gekennzeichnet, daß das rekombinante Protein eine Leitsequenz für die Ausschleusung des Proteins ins Periplasma enthält.

9. Verfahren gemäß mindestens einem der Ansprüche 1 bis 8, dadurch gekennzeichnet, daß das rekombinante Protein unter der Kontrolle eines Promoters, wie lac-, tac-, T7 steht.

10. Verfahren gemäß mindestens einem der Ansprüche 1 bis 9, dadurch gekennzeichnet, das das Plasmid im Falle der bakteriellen Expression ein pBM1.1-Derivat ist.

11. Verfahren gemäß mindestens einem der Ansprüche 1 bis 10, dadurch gekennzeichnet, daß das rekombinante Protein ein Immuntoxin oder ein chimäres Protein mit bevorzugt mehreren Bindungsfunktionen ist.

12. Verfahren gemäß einem der Ansprüche 1 bis 11, dadurch gekennzeichnet, daß die Expression des kompatiblen Soluts zusätzlich über ein kotransformiertes Plasmid, insbesondere ein pOSM-Derivat gesteuert wird.

13. Verfahren gemäß einem der Ansprüche 1 bis 12, dadurch gekennzeichnet, daß das Operon für die Expression des kompatiblen Solutes zusätzlich auf einem pBM1.1-Derivat oder das Operon des rekombinanten Proteins auf pOSM codiert wird.

14. Verfahren gemäß einem der Ansprüche 1 bis 13, dadurch gekennzeichnet, daß die Expression des rekombinanten Proteins bei Temperaturen von 21-28°C durchgeführt wird.

15. Verfahren zur Gewinnung rekombinanter Proteine, dadurch gekennzeichnet, daß die transformierten Organismen nach Expression gemäß mindestens einem der Ansprüche 1 bis 14, beispielsweise durch Verbringung der transformierten Organismen in Umgebungsbedingungen geringerer Salz- und/oder Zuckerkonzentrationen, oder durch Einfrieren und Auftauten und/oder Ultraschall und/oder French Press, vorzugsweise bei 4°C aufgeschlossen und die Proteine isoliert werden.

16. Verfahren gemäß Anspruch 15, dadurch gekennzeichnet, daß die rekombinanten Proteine nach Expression und Aufschluß durch chromatografische Verfahren bis zur Homogenität gereinigt werden.
